# EUROPEAN PATENT SPECIFICATION

(11) **EP 4 430 401 B1**
(45) Date of publication and mention of the grant of the patent: **30.04.2025**
(21) Application number: 22817115.3
(22) Date of filing: 07.11.2022
(51) Int. Cl.: G01N 33/574, C07K 16/00

(54) **COLLAGEN TYPE XXII ASSAY**
KOLLAGEN-TYP-XXII-TEST
DOSAGE DE COLLAGÈNE DE TYPE XXII

(30) Priority: 08.11.2021 GB 202116008
(43) Date of publication of application: 18.09.2024
(73) Proprietor: Nordic Bioscience A/S, 2730 Herlev (DK)
(72) Inventor: MADSEN, Emilie Albrecht, 2400 København NV (DK); THORLACIUS-USSING, Jeppe, 3460 Birkerød (DK); WILLUMSEN, Nicholas, 2820 Gentofte (DK); KARSDAL, Morten Asser, 2100 København Ø (DK)
(74) Representative: Beck Greener LLP
(86) International application number: PCT/EP2022/080910
(87) International publication number: WO 2023/079130

(56) References cited:
- MISAWA KIYOSHI ET AL: "Prognostic value of type XXII and XXIV collagen mRNA expression in head and neck cancer patients", MOLECULAR AND CLINICAL ONCOLOGY, vol. 2, no. 2, 1 March 2014 (2014-03-01), GR, pages 285 - 291, XP093022757, ISSN: 2049-9450, Retrieved from the Internet <URL:https://www.ncbi.nlm.nih.gov/pmc/articles/PMC3917764/pdf/mco-02-02-0285.pdf> DOI: 10.3892/mco.2013.233
- MADSEN EMILIE A. ET AL: "Type XXII Collagen Complements Fibrillar Collagens in the Serological Assessment of Tumor Fibrosis and the Outcome in Pancreatic Cancer", CELLS, vol. 11, no. 23, 24 November 2022 (2022-11-24), pages 3763, XP093022417, DOI: 10.3390/cells11233763

## Description

### Field of the Invention

The present invention relates to immunoassays, in particular immunoassays for detecting and/or monitoring cancer in a patient, and to monoclonal antibodies and immunoassay kits for use in carrying out said immunoassays.

### Background

Cancer is a major global health problem and is estimated to cause more than 600.000 cancer deaths in 2021 in the United States alone[1]. Despite continuous research and progress, the cancer burden persists. One immediate need for cancer patients is better non-invasive diagnostic and prognostic biomarkers. Contrary to traditional tissue-based approaches, blood-based biomarkers do not require tissue biopsies and are less invasive, resulting in less discomfort and fewer complications[2],[3].

The tumor microenvironment is intricately involved in cancer development and was recently recognized alongside the traditional hallmarks of cancer[4]. The tumor microenvironment comprises the cancer cells and the surrounding stroma. Within the stroma are various cells of the immune system and fibroblasts. Fibroblasts contribute to production of collagens that are key components of the large non-cellular component of most tissues, the extracellular matrix (ECM). The ECM can accumulate around a tumor, forming a surrounding layer of matrix that encapsulates the growth, and can inhibit the ability of immune cells or drugs to challenge the cancer[5]. In healthy tissue, ECM components are constantly produced and degraded in a delicate balance. In cancer, however, this balance in turnover is skewed[6],[7].

Collagens, of which 28 have been identified to date, are the major constituents of the ECM along with laminins, proteoglycans, and glycoproteins. The collagens span the ECM from the basement membrane (BM), at the basal side of epithelial and endothelial cells and into the interstitial matrix (IM). At the basement membrane zones, in the interface between the BM and IM, are some of the so-called minor collagens such as the FACITs (fibril-associated collagens with interrupted triple helices). It has been proposed that the FACIT family of collagens function as bridges, as they mediate interactions between collagen fibrils and other ECM components. In this way they assist with organization and stability of the ECM[7],[8]. Collagen XXII (COL22) is a minor collagen and a member of the FACIT family of collagens. COL22 is known to be expressed at sites of tissue junctions in muscle, cartilage, heart, skin and associated with maintaining vascular stability[9],[10]. COL22 also has prognostic value in squamous cell carcinoma of the head and neck (HNSCC) where COL22A1 mRNA expression was significantly increased in cases with lymph node metastasis. Moreover, mRNA expression levels were significantly associated with earlier disease recurrence and upregulation of COL22A1 mRNA was associated with a decrease in disease free survival[11]. Separately, COL22 was identified in a human *ex-vivo* and *in vitro* model as an early response gene to transforming growth factor beta using both skin- and lung-derived cells (including adenocarcinomic human alveolar basal epithelial cells, A549 cells). This induction of COL22A1 was significantly increased on both mRNA and protein levels[12]. It has been observed for several FACITs that they are more pronounced during development and/or tissue repair, among these are type XVI, XX and XXII collagens [13],[14],[15].

The role of COL22 in cancer was investigated by the inventors, as several parallels have been suggested to exist between cancer and development, as well as tissue repair [16],[17].

### Summary

The applicant has now developed an enzyme-linked immunosorbent assay (ELISA) to quantify the presence of a biomarker (referred to herein as "PRO-C22") of type XXII collagen, in blood. The assay was optimized and validated, and used to determine the levels of circulating type XXII collagen in the serum of cancer patients and healthy controls. It was found that the assay was robust, with specificity for type XXII collagen and with sensitivity to detect levels in both healthy controls and patients with disease (cancer). The data shows that PRO-C22 levels were significantly elevated in the serum of cancer patients compared to healthy controls.

Accordingly, in a first aspect, the present invention provides a method of immunoassay, the method comprising:
(i) contacting a sample from a patient with a monoclonal antibody that specifically binds to a C-terminus amino acid sequence AARPGNVKGP (SEQ ID No. 1) (which C-terminus amino acid sequence is referred to herein as "PRO-C22" and/or the "target sequence", peptides consisting of or comprising said C-terminus amino acid sequence also being referred to herein as "PRO-C22" and/or the "target peptide"); and
(ii) detecting and determining the amount of binding between said monoclonal antibody and peptides in the sample.

The method is preferably a method of immunoassay for detecting and/or monitoring a disease in a patient. The method preferably further comprises:
(iii) correlating said amount of binding of said monoclonal antibody as determined in step (ii) with values associated with normal healthy subjects, and/or with values associated with known disease severity, and/or with values obtained from said patient at a previous time point, and/or with a predetermined cut-off value.

In preferred embodiments, the disease is cancer. The cancer may for example be bladder cancer, breast cancer, colorectal cancer, head and neck cancer, kidney cancer, lung cancer, melanoma, ovarian cancer, pancreatic cancer, prostate cancer, or stomach cancer. Preferably the cancer is pancreatic cancer.

If the patient has pancreatic cancer, the method can be used to provide a prognosis of overall survival. Thus the method may further comprise providing a prognosis of overall survival, wherein an amount of binding of said monoclonal antibody as determined in step (ii)above the median (50^{th} percentile) value associated with known pancreatic cancer patients is associated with poor overall survival. An amount of binding below the median (50^{th} percentile) is associated with improved overall survival.

In preferred embodiments, the monoclonal antibody does not specifically bind to an elongated version of the target sequence which is AARPGNVKGPX (SEQ ID No. 2) where X is any amino acid (i.e. a version of the PRO-C22 target sequence extended at its C-terminus by the addition of an amino acid). Preferably X is A and the elongated version of the target sequence which is AARPGNVKGPA (SEQ ID No. 3) (i.e. a version of the PRO-C22 target sequence extended at its C-terminus by the addition of alanine). Preferably, the ratio of the affinity of said antibody for the PRO-C22 target sequence to the affinity of said antibody for the elongated version of the target sequence is at least 10 to 1, and more preferably is at least 20 to 1 or at least 30 to 1.

In preferred embodiments, the monoclonal antibody does not specifically bind to a truncated version of the target sequence which is AARPGNVKG (SEQ ID No. 4) (i.e. a version of the PRO-C22 target sequence truncated by removal of the final proline residue). Preferably, the ratio of the affinity of said antibody for the PRO-C22 target sequence to the affinity of said antibody for the truncated version of the target sequence is at least 10 to 1, and more preferably is at least 20 to 1 or at least 30 to 1.

Preferably, the monoclonal antibody is a monoclonal antibody that is raised against a synthetic peptide having the C-terminus amino acid sequence AARPGNVKGP (SEQ ID No. 1).

Once the disease has been detected in the patient, the method may further comprise the step of administering a suitable treatment to the patient. For example if the disease is cancer, a known treatment for that cancer can be administered such as chemotherapy, radiotherapy, hormone therapy, immunotherapy, stem cell transplant, surgery, targeted therapy or a combination thereof.

The sample is preferably a biofluid. The biofluid may be, but is not limited to, blood, serum, plasma, urine or a supernatant from cell or tissue cultures. Preferably the biofluid is blood, serum or plasma.

The immunoassay may be, but is not limited to, a competition assay or a sandwich assay. The immunoassay may, for example, be a radioimmunoassay or an enzyme-linked immunosorbent assay (ELISA). Such assays are techniques known to the person skilled in the art.

As used herein the term "ELISA" (enzyme-linked immunosorbent assay) refers to an immunoassay in which the target peptide present in a sample (if any) is detected using antibodies linked to an enzyme, such as horseradish peroxidase or alkaline phosphatase. The activity of the enzyme is then assessed by incubation with a substrate generating a measurable product. The presence and/or amount of target peptide in a sample can thereby be detected and/or quantified. ELISA is a technique known to those skilled in the art.

As used herein the term, the term "competitive ELISA" refers to a competitive enzyme-linked immunosorbent assay. In a "competitive ELISA" the target peptide present in a sample (if any) competes with known amount of target of peptide (which for example is bound to a fixed substrate or is labelled) for to binding an antibody, and is a technique known to the person skilled in the art.

As used herein the term "sandwich immunoassay" refers to the use of at least two antibodies for the detection of an antigen in a sample, and is a technique known to the person skilled in the art.

As used herein the term "amount of binding" refers to the quantification of binding between the monoclonal antibody and peptides in the patient sample. Said quantification may for example be determined by comparing the measured values of binding in the patient sample against a calibration curve produced using measured values of binding in standard samples containing known concentrations of a peptide to which the antibody specifically binds, in order to thereby determine the quantity of peptide to which the antibody specifically binds in the patient sample. In the Examples set out below, an ELISA method is used in which spectrophotometric analysis is used to measure the amount of binding both in the patient samples and when producing the calibration curve. However, any suitable analytical method can be used.

As used herein the term "predetermined cut-off value" means an amount of binding that is determined statistically to be indicative of a high likelihood of a disease or a particular severity thereof in a patient, in that a measured value of the target peptide in a patient sample that is at or above the statistical cut-off value corresponds to at least a 70% probability, preferably at least an 75% probability, more preferably at least an 80% probability, more preferably at least an 85% probability, more preferably at least a 90% probability, and most preferably at least a 95% probability of the presence of said disease or said particular severity thereof.

As used herein, the term "values associated with normal healthy subjects" means standardised quantities of binding determined by the method described supra for samples from subjects considered to be healthy, i.e. without disease; and the term "values associated with known disease severity or prognosis" means standardised quantities of binding determined by the method described supra for samples from patients known to have disease of a known severity.

As used herein the term "C-terminus" refers to a C-terminal peptide sequence at the extremity of a polypeptide, i.e. at the C-terminal end of the polypeptide, and is not to be construed as meaning in the general direction thereof. As used herein, the terms "peptide" and "polypeptide" are used synonymously.

As used herein the term "monoclonal antibody" refers to both whole antibodies and to fragments thereof that retain the binding specificity of the whole antibody, such as for example a Fab fragment, F(ab')2 fragment, single chain Fv fragment, or other such fragments known to those skilled in the art. As is well known, whole antibodies typically have a "Y-shaped" structure of two identical pairs of polypeptide chains, each pair made up of one "light" and one "heavy" chain. The N-terminal regions of each light chain and heavy chain contain the variable region, while the C-terminal portions of each of the heavy and light chains make up the constant region. The variable region comprises three complementarity determining regions (CDRs), which are primarily responsible for antigen recognition. The constant region allows the antibody to recruit cells and molecules of the immune system. Antibody fragments retaining binding specificity comprise at least the CDRs and sufficient parts of the rest of the variable region to retain said binding specificity.

In the methods of the present invention, a monoclonal antibody comprising any constant region known in the art can be used. Human constant light chains are classified as kappa and lambda light chains. Heavy constant chains are classified as mu, delta, gamma, alpha, or epsilon, and define the antibody's isotype as IgM, IgD, IgG, IgA, and IgE, respectively. The IgG isotype has several subclasses, including, but not limited to IgGl, IgG2, IgG3, and IgG4. The monoclonal antibody may preferably be of the IgG isotype, including any one of IgGl, IgG2, IgG3 or IgG4.

The CDR of an antibody can be determined using methods known in the art such as that described by Kabat et al[18]. Antibodies can be generated from B cell clones as described in the examples. The isotype of the antibody can be determined by ELISA specific for human IgM, IgG or IgA isotype, or human IgG1, IgG2, IgG3 or IgG4 subclasses. The amino acid sequence of the antibodies generated can be determined using standard techniques. For example, RNA can be isolated from the cells, and used to generate cDNA by reverse transcription. The cDNA is then subjected to PCR using primers which amplify the heavy and light chains of the antibody. For example primers specific for the leader sequence for all VH (variable heavy chain) sequences can be used together with primers that bind to a sequence located in the constant region of the isotype which has been previously determined. The light chain can be amplified using primers which bind to the 3' end of the Kappa or Lamda chain together with primers which anneal to the V kappa or V lambda leader sequence. The full length heavy and light chains can be generated and sequenced.

In a second aspect, the present invention provides a monoclonal antibody that specifically binds to a C-terminus amino acid sequence AARPGNVKGP (SEQ ID No. 1). The monoclonal antibody is suitable for use in an immunoassay according to the first aspect of the invention, and preferred and other optional embodiments of the monoclonal antibody according to the second aspect of the invention will be apparent from the discussion *supra* of the monoclonal antibodies for use the in the first aspect of the invention and preferred and other optional embodiments thereof.

Monoclonal antibodies that specifically bind to the C-terminus amino acid sequence AARPGNVKGP (SEQ ID No. 1) can be generated via any suitable techniques known in the art. For example, the monoclonal antibody may be raised against a synthetic peptide having the amino acid sequence AARPGNVKGP (SEQ ID No. 1), such as for example by: immunizing a rodent (or other suitable mammal) with a synthetic peptide consisting of the sequence AARPGNVKGP (SEQ ID No. 1), which optionally may linked to an immunogenic carrier protein (such as keyhole limpet hemocyanin), isolating and cloning a single antibody producing cell, and assaying the resulting monoclonal antibodies to ensure that they have the desired specificity. An exemplary protocol for producing a monoclonal antibody that that specifically bind to the C-terminus amino acid sequence AARPGNVKGP (SEQ ID No. 1) is described infra.

Preferably, the monoclonal antibody or fragment thereof may preferably comprise one or more complementarity-determining regions (CDRs) selected from:

| | |
|---|---|
| CDR-L1: | KASQDIYSSLS (SEQ ID No. 5) |
| CDR-L2: | RANRLIN (SEQ ID No. 6) and |
| CDR-L3: | LQYDDFPYM (SEQ ID No. 7) |
| CDR-H1: | TYGVH (SEQ ID No. 8) |
| CDR-H2: | AIWRGGSTDYNPAFMS (SEQ ID No. 9) and |
| CDR-H3: | RSTLFYFDY (SEQ ID No. 10) |

Preferably the antibody or fragment thereof comprises at least 2, 3, 4, 5 or 6 of the above listed CDR sequences.

Preferably the monoclonal antibody or fragment thereof has a light chain variable region comprising the CDR sequences

| | |
|---|---|
| CDR-L1: | KASQDIYSSLS (SEQ ID No. 5) |
| CDR-L2: | RANRLIN (SEQ ID No. 6) and |
| CDR-L3: | LQYDDFPYM (SEQ ID No. 7) |

Preferably the monoclonal antibody or fragment thereof has a light chain that comprises framework sequences between the CDRs, wherein said framework sequences are substantially identical or substantially similar to the framework sequences between the CDRs in the light chain sequence below (in which the CDRs are shown in bold and underlined, and the framework sequences are shown in italics)

KASQDIYSSLSWFQQKPGKSPKTLIYRANRLINGVPSRFSGSGSGQDYSLTISSLEYEDVGI
YYCLQYDDFPYM (SEQ ID No. 11)

Preferably the monoclonal antibody or fragment thereof has a heavy chain variable region comprising the CDR sequences

| | |
|---|---|
| CDR-H1: | TYGVH (SEQ ID No. 8) |
| CDR-H2: | AIWRGGSTDYNPAFMS (SEQ ID No. 9) and |
| CDR-H3: | RSTLFYFDY (SEQ ID No. 10) |

Preferably the monoclonal antibody or fragment thereof has a heavy chain that comprises framework sequences between the CDRs, wherein said framework sequences are substantially identical or substantially similar to the framework sequences between the CDRs in the heavy chain sequence below (in which the CDRs are shown in bold and underlined, and the framework sequences are shown in italics)

TYGVHWIRQSPGKGLEWLGAIWRGGSTDYNPAFMSRLSITKDISKSQVFFKMNSLQADDTAI
YYCAKRSTLFYFDY (SEQ ID No. 12)

As used herein, the framework amino acid sequences between the CDRs of an antibody are substantially identical or substantially similar to the framework amino acid sequences between the CDRs of another antibody if they have at least 70%, 80%, 90% or at least 95% similarity or identity. The similar or identical amino acids may be contiguous or non-contiguous.

The framework sequences may contain one or more amino acid substitutions, insertions and/or deletions. Amino acid substitutions may be conservative, by which it is meant the substituted amino acid has similar chemical properties to the original amino acid. A skilled person would understand which amino acids share similar chemical properties. For example, the following groups of amino acids share similar chemical properties such as size, charge and polarity: Group 1 Ala, Ser, Thr, Pro, Gly; Group 2 Asp, Asn, Glu, Gln; Group 3 His, Arg, Lys; Group 4 Met, Leu, Ile, Val, Cys; Group 5 Phe Thy Trp.

A program such as the CLUSTAL program to can be used to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention. Identity or similarity is preferably calculated over the entire length of the framework sequences.

In certain preferred embodiments, the monoclonal antibody or fragment thereof may comprise the light chain variable region sequence:
*DIKMTQSPSSMHASLGERVTITC***KASQDIYSSLS***WFQQKPGKSPKTLIY***RANRLIN***GVPSRF SGSGSGQDYSLTISSLEYEDVGIYYC***LQYDDFPYM***FGGGTKLEIK* (SEQ ID No. 13) (CDRs bold and underlined; Framework sequences in italics) and/or the heavy chain variable region sequence:
*QVQLKQSGPSLVQPSQSLSITCTVSGFSLT***TYGVH***WIRQSPGKGLEWLG***AIWRGGSTDYNPA FMS***RLSITKDISKSQVFFKMNSLQADDTAIYYCAK***RSTLFYFDY***WGQGTTLTVS* (SEQ ID No. 14) (CDRs bold and underlined; Framework sequences in italics)

In a third aspect, the application relates to an immunoassay kit comprising a monoclonal antibody according to the second aspect, and at least one of;
- a streptavidin coated well plate;
- a biotinylated peptide Biotin-L-AARPGNVKGP (SEQ ID No. 15), wherein L is an optional linker;
- a secondary antibody for use in a sandwich immunoassay;
- a calibrator protein comprising the C-terminus amino acid sequence AARPGNVKGP (SEQ ID No. 1);
- an antibody biotinylation kit;
- an antibody HRP labelling kit;
- an antibody radiolabelling kit; and
- an assay visualisation kit.

The invention will now be described in the examples which reference the following figures:

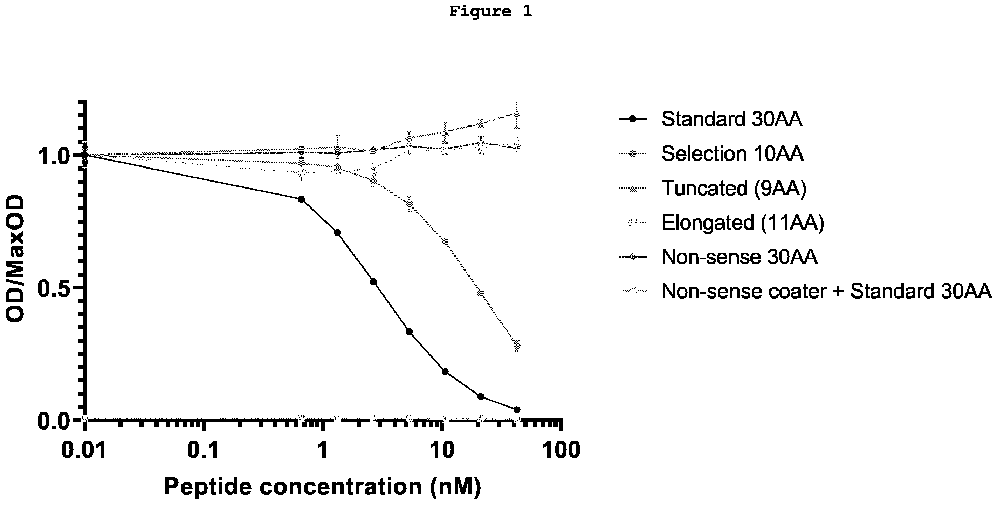

Figure 1 shows specificity of PRO-C22 ELISA. Dose dependent inhibition of biotinylated coating peptide (Biotin-AARPGNVKGP (SEQ ID No. 15)) and monoclonal antibody interaction by the assay specific standard peptides respectively 30 (GPPGPPGQCDPSQCAYFASLAARPGNVKGP (SEQ ID No. 16)) & 10 (AARPGNVKGP (SEQ ID No. 1)) amino acid (AA) residues. No inhibition was observed when truncating (AARPGNVKG (SEQ ID No. 4)) or elongating (AARPGNVKGPA (SEQ ID No. 3)) the peptide sequence from the C-terminus. A non-sense 30 AA peptide PGMRGMPGSPGGPGSDGKPGPPGSQGESGR (SEQ ID No. 17) had no inhibitory effect and addition of a non-sense coating peptide PPGSQGESGR-Biotin (SEQ ID No. 18) combined with the assay specific 30 AA standard peptide exhibited no effect regarding interaction. Error bars indicate standard PRO-C22 in serum of cancer patients.

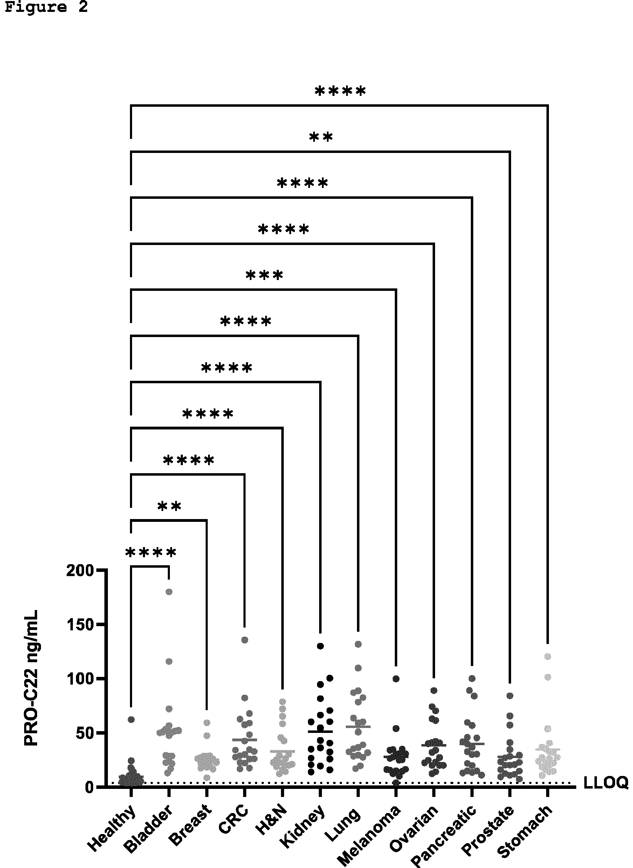

Figure 2 shows quantification of PRO-C22 in the serum of healthy controls (n = 33) and bladder cancer (n = 20), breast cancer (n = 20), colorectal cancer (n = 20), head & neck cancer (n = 20), kidney cancer (n = 20), lung cancer (n = 20), melanoma (n = 20), ovarian cancer (n = 20), pancreatic cancer (n = 20), prostate cancer (n = 20) and stomach cancer (n = 20). PRO-C22 levels are presented individually with bars marking the mean as scatter plots. Samples measuring below the LLOQ were given the LLOQ value determined in the validation of PRO-C22. **** indicates a p-value below 0.0001. *** below 0.001. ** below 0.01.

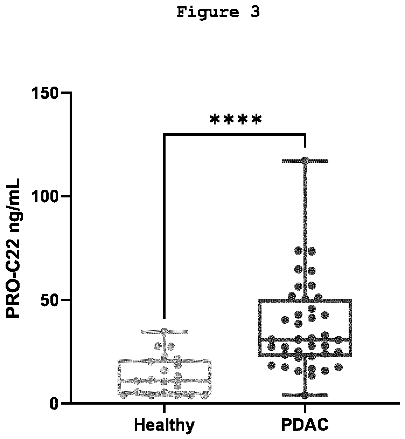

Figure 3 shows - PRO-C22 levels measured in serum from patients enrolled in the BIOPAC cohort. (Left) PDAC patients (n=39) levels of PRO-C22 were significantly elevated compared to healthy controls (n=20).

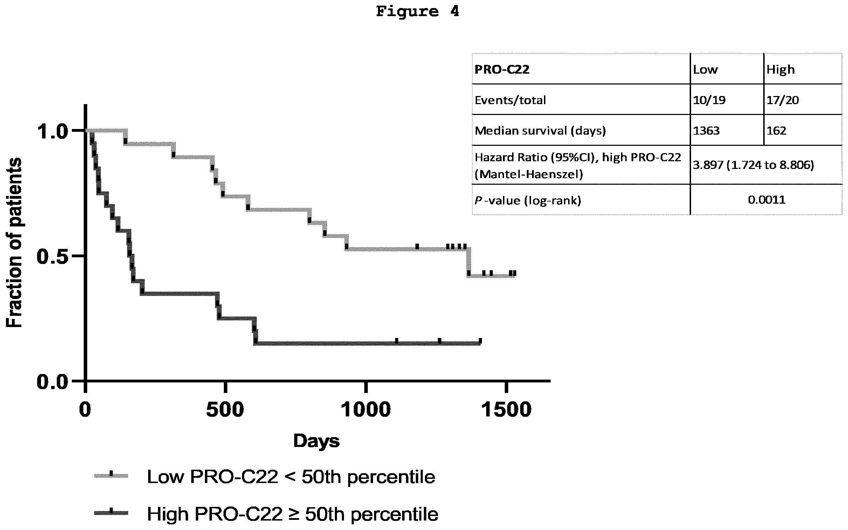

Figure 4 shows that high PRO-C22 (above the median: 50th percentile) is significantly associated with poor overall survival in pancreas cancer.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Materials and Methods

Generation of Monoclonal Antibodies Targeting PRO-C22 A 10 amino-acid peptide ¹⁶¹⁶AARPGNVKGP¹⁶²⁶ (SEQ ID No. 1) corresponding to the C-terminus of type XXII collagen (UniprotKB: Q8NFW1) was purchased from Genscript (Piscataway, NJ, USA) and used for immunization.

Six-week-old Balb/C female mice were subcutaneously injected with emulsified KLH-CGG-conjugated immunogenic peptide (KLH-CGG-AARPGNVKGP (SEQ ID No. 19)). This was generated by covalently cross-linking the target peptide to Keyhole Limpet Hemocyanin (KLH) carrier protein using sulfosuccinimidyl 4-(N-maleimidomethyl) cyclohexane-1-carboxylate, SMCC (Thermo Scientific, Waltham, MA, USA, cat.no. 22322). Glycine and cysteine residues were added at the N-terminal end to ensure right linking of the carrier protein. Immunizations using 200 µL emulsified antigen containing 100 µg immunogenic peptide mixed with Sigma Adjuvant System (Sigma cat. No. S6322), were performed at 2-week intervals until stable sera titer levels were reached. The mouse with the highest titer was rested for four weeks and was then boosted with 100 µg immunogenic peptide in 100 µL 0.9% NaCl solution intravenously and terminated.

Hybridoma cells were produced by fusing spleen cells with SP2/0 myeloma cells as previously described (Gefter, Margulies and Scharff, 1977). The resultant hybridoma cells were then cultured in 96-well microtiter plates and subcloning was done while limiting dilution of cells. The selected monoclonal cells were seeded in 24-well plates and then expanded in T25, then T75 and finally in T150 flasks before collection of supernatants. Approximately 500mL was collected and purified using protein-G-columns according to the manufacturer's instructions (GE Healthcare Life Sciences, Little Chalfont, UK, cat. #17-0404-01).

The antibody generated was sequenced and the CDRs determined. Total RNA was isolated from the hybridoma cells following the technical manual of RNA-easy Isolation Reagent. Total RNA was then reverse transcribed into cDNA using isotype-specific anti-sense primers or universal primers following the technical manual of SMARTScribe Reverse Transcriptase. The antibody fragments of VH and VL were amplified according to the standard operating procedure (SOP) of rapid amplification of cDNA ends (RACE) of GenScript. Amplified antibody fragments were cloned into a standard cloning vector separately. Colony PCR was performed to screen for clones with inserts of correct sizes. No less than five colonies with inserts of correct sizes were sequenced for each fragment. The sequences of different clones were aligned and the consensus sequence of these clones was provided.

The sequence of the chains are as follows (CDRs in bold; Framework sequence in Italics; Constant region underlined):
Heavy chain: Amino acid sequence (276 aa) (Mouse IgG2 isotype:

QVQLKQSGPSLVQPSQSLSITCTVSGFSLTTYGVHWIRQSPGKGLEWLGAIWRGGSTDYNPA
FMSRLSITKDISKSQVFFKMNSLQADDTAIYYCAKRSTLFYFDYWGQGTTLTVSSAKTTPPS
VYPLAPGCGDTTGSSVTLGCLVKGYFPESVTVTWNSGSLSSSVHTFPALLQSGLYTMSSSVT
VPSSTGQVRPSPAALLTQPAAPRWTKNLSPAGPFQQSTPVLHARSVTNAQLLTSRVDHPSSS
S LQISRMYS (SEQ ID No. 20)

| | |
|---|---|
| CDR-H1: | TYGVH (SEQ ID No. 8) |
| CDR-H2: | AIWRGGSTDYNPAFMS (SEQ ID No. 9) and |
| CDR-H3: | RSTLFYFDY (SEQ ID No. 10) |

Light chain: Amino acid sequence (234 aa) (mouse Kappa isotype)

DIKMTQSPSSMHASLGERVTITCKASQDIYSSLSWFQQKPGKSPKTLIYRANRLINGVPSRF
SGSGSGQDYSLTISSLEYEDVGIYYCLQYDDFPYMFGGGTKLEIKRADAAPTVSIFPPSSEQ
LTSGGASVVCFLNNFYPKDINVKWKIDGSERQNGVLNSWTDQDSKDSTYSMSSTLTLTKDEYE
RHNSYTCEATHKTSTSPIVKSFNRNEC (SEQ ID No. 21)

| | |
|---|---|
| CDR-L1: | KASQDIYSSLS (SEQ ID No. 5) |
| CDR-L2: | RANRLIN (SEQ ID No. 6) and |
| CDR-L3: | LQYDDFPYM (SEQ ID No. 7) |

### PRO-C22 ELISA Protocol

The final PRO-C22 protocol was performed as follows: a 96-well streptavidin-coated ELISA plate was coated with 100 µL/well of 20 ng/mL biotinylated AARPGNVKGP (Biotin-AARPGNVKGP (SEQ ID No. 15)) peptide dissolved in assay buffer (25 mM TBS, 1% BSA (w/v), 0.1% Tween-20 (w/v), 2 g/L NaCl, pH 8.0) and incubated for 30 minutes at 20 °C with shaking at 300 RPM. After washing five times with washing buffer (25 mM Tris, 50 mM NaCl, pH 7.2), 20 µL/well of sample was added in duplicates followed by 100 µL/well of 35 ng/mL HRP-labelled monoclonal antibody in assay buffer and incubated for 20 hours at 4 °C with shaking at 300 RPM. A second washing cycle was performed and 100 µL/well of 3,3',5,5'-Tetramethylbenzidine (TMB) was added and incubated for 15 minutes in darkness at 20 °C with shaking at 300 RPM. The reaction was stopped by adding 100 µL/well of 1% H₂SO₄. Absorbance was measured at 450 nm with 650 nm as reference. A standard curve was generated using 20 µL/well of 125 ng/mL GPPGPPGQCDPSQCAYFASLAARPGNVKGP (SEQ ID No. 16) standard peptide, serially diluted two-fold. A four-parametric logistic regression model was used to fit a curve. Each plate included two kit controls of peptide-in-assay-buffer, and three quality control samples comprising one human serum sample, one pig serum sample and one synovial fluid sample.

### Technical validation of the PRO-C22 ELISA

The specificity of the antibody was evaluated by signal inhibition. Using two-fold dilutions of two versions of the selection peptide, a ten amino acid (AA) version (AARPGNVKGP (SEQ ID No. 1)) and a 30 AA version
(GPPGPPGQCDPSQCAYFASLAARPGNVKGP (SEQ ID No. 16)) and comparing those to a truncated (AARPGNVKG (SEQ ID No. 4)), elongated (AARPGNVKGPA (SEQ ID No. 3)) as well as a non-sense biotinylated peptide (PGMRGMPGSP-Biotin (SEQ ID No. 22)) and non-sense standard peptide (PGMRGMPGSPGGPGSDGKPGPPGSQGESGR (SEQ ID No. 17)) was tested.

The lower- and upper limit of measurement range (LLMR and ULMR), defined as the concentration limits of the linear range of the assay, was determined across ten independent runs. From these runs the inter- and intra-assay variation were determined based on ten samples covering the LLMR-ULMR measurement range. The ten samples comprised three human serum samples, two peptide-in-assay-buffer, in addition to the two kit controls and three quality control samples. The intra-assay variation was calculated as the mean coefficient of variance (CV%) within plates. The inter-assay variation was based on the mean CV% between plates. From the ten runs, the upper limit of quantification (ULOQ) was determined as the highest accepted standard point based on the recovery percentage (RE%) and CV% of this. The criteria of acceptance was CV% ≤ 20 % and %RE within 80-120%. The lower limit of quantification (LLOQ) was determined as the concentration at which CV% goes above 20% for most samples based on four human serum samples with low analyte concentrations that were measured in sextuplicates over five independent runs.

Linearity or parallelism was tested by serially diluting four healthy human serum samples two-fold and calculating the CV% through three independent runs. The RE% was calculated relative to the 1:2 diluted values. The acceptance criteria of the CV% was ≤ 20 % and the RE% within 80-120%.

Accuracy was evaluated by spiking 30AA standard peptide into three human serum samples and calculating the RE%. Acceptance criteria of the mean RE% was 80-120%. The accuracy was further evaluated by mixing three sets of serum samples in different amounts (e.g., 25% serum A + 75% serum B, 50% serum A + 50% serum B, etc.) and calculating the RE% to the individual reference serum sample values. RE% of each spiking ratio should be within 80-120%. Effect of commonly interfering substances biotin, lipids and hemoglobin was tested using three serum samples and by different concentrations (biotin low: 5ng/mL, high: 100ng/mL; lipids low: 1.5mg/mL, high: 5mg/mL; hemoglobin: ten concentrations ranging from 0.5mg/mL to 5.0mg/mL, varying by 0.5mg/mL). Interference was evaluated by RE% and accepted within 80-120%.

Analyte stability was assessed by incubating aliquots of three serum samples for 2, 4, 24 or 48 hrs at both 4°C and 20°C. The RE% was calculated relative to control serum samples, thawed upon analysis, and RE% was accepted within 80-120%. Stability was further evaluated through repeated freeze-thaw cycles of serum samples: up to five rounds of freeze-thaw cycles. Freeze-thaw stability was calculated based on the RE% relative to samples that underwent a single thawing prior to analysis, accepted if RE% was within 80-120%. The technical stability of the assay was evaluated by three runs of the assay using kit reagents incubated at 20°C for 24 hrs and calculating the RE% of ten samples measured using reagents from frozen storage, acceptance criteria of RE% within 80-120%.

### Patient samples

The first cohort included 223 cancer samples and 33 healthy samples. It included 20 patients each of pancreatic-, colorectal-, kidney-, stomach-, breast-, bladder-, lung-, melanoma-, head and neck-, prostate- and ovarian cancer patients and 33 age matched healthy controls. All cancer samples were obtained from Proteogenex (Los Angeles, CA, USA) and the healthy controls were obtained from BioIVT (Westbury, NY, USA). A summary of the first cohort characteristics can be found in Table 1.

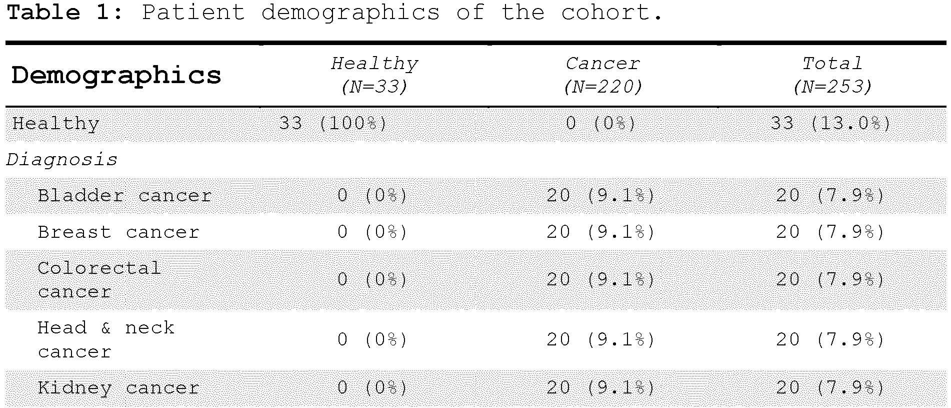

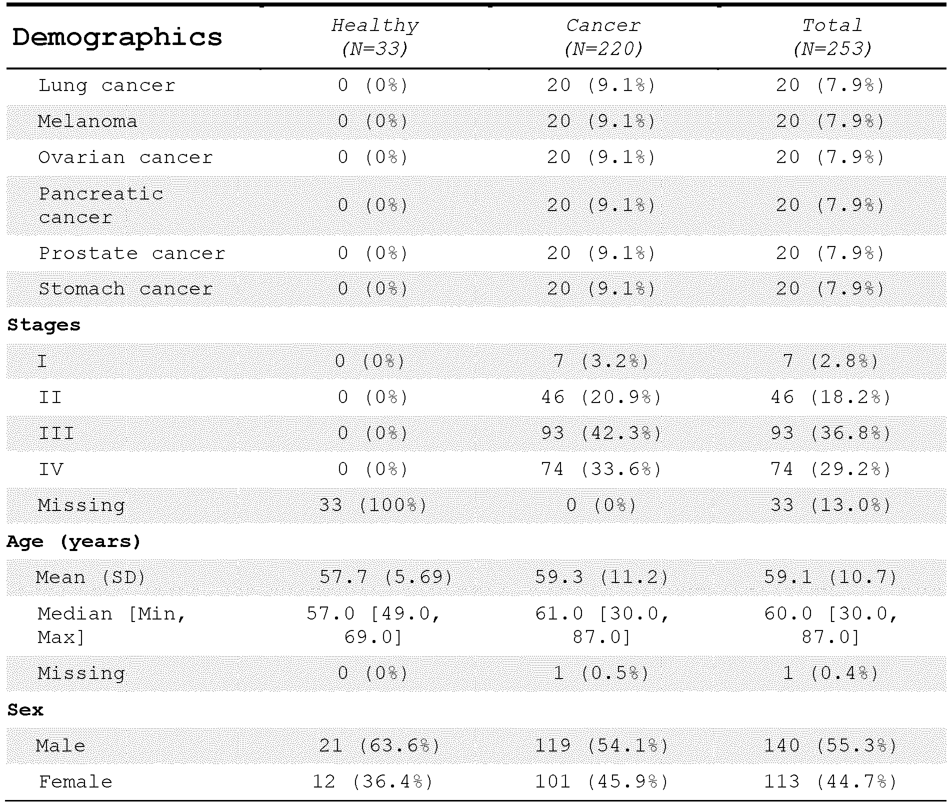

The second cohort included 20 healthy donors and 39 patients with pancreas ductal adenocarcinoma (PDAC). All patients were from the Danish BIOPAC study "BIOmarkers in patients with Pancreatic Cancer" (NCT03311776). Patients were recruited from six Danish hospitals from December 2008 until September 2017. PC patients had histologically confirmed tumors. The PC patients were treated with various types of chemotherapy according to national guidelines (www.gicancer.dk). The study was carried out in accordance with the recommendations of the Danish Regional Committee on Health Research Ethics. The BIOPAC protocol was approved by the Danish Regional Committee on Health Research Ethics (VEK ref. KA-20060113) and the Data Protection Agency (j.nr. 2006-41-6848). Blood samples were obtained at the time of diagnosis or before operation. All subjects gave written informed consent in accordance with the Declaration of Helsinki.

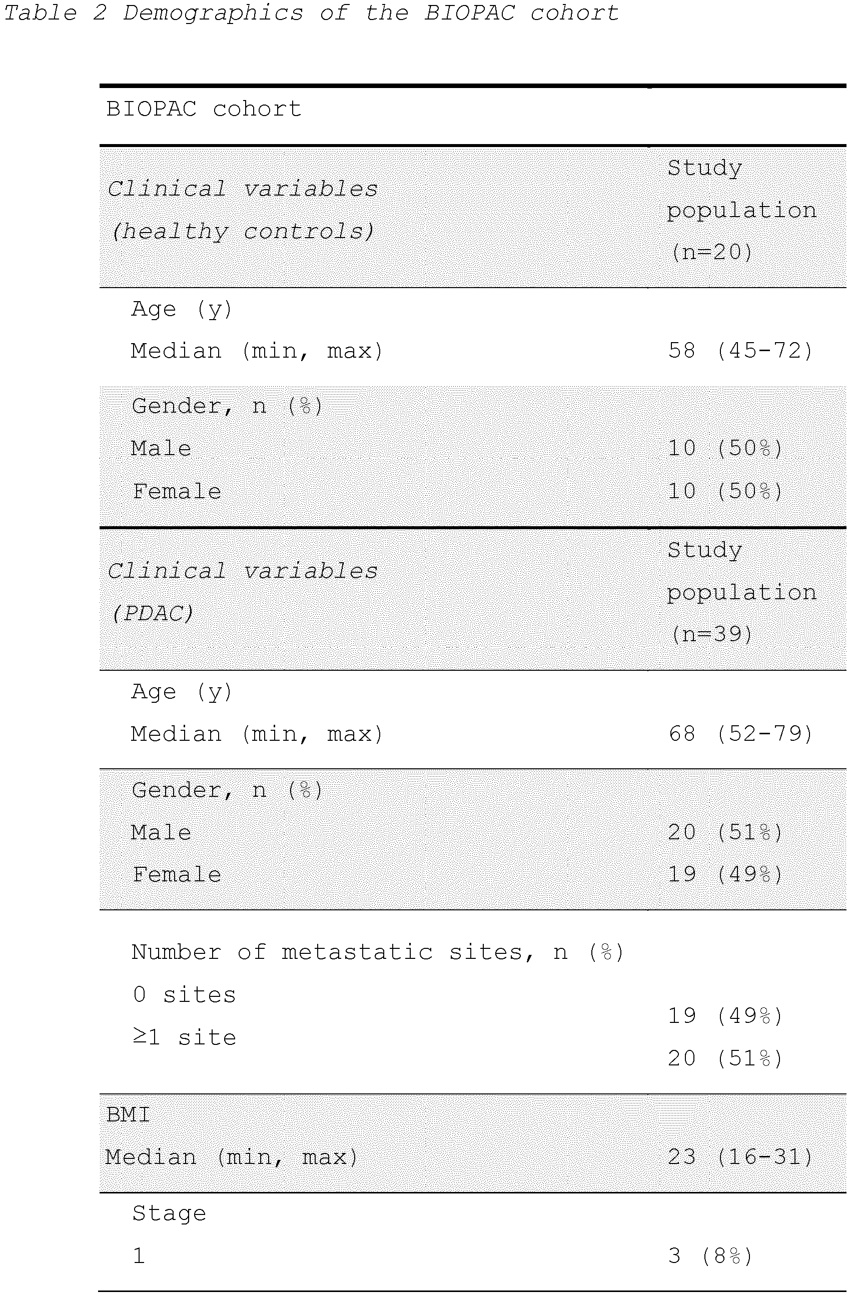

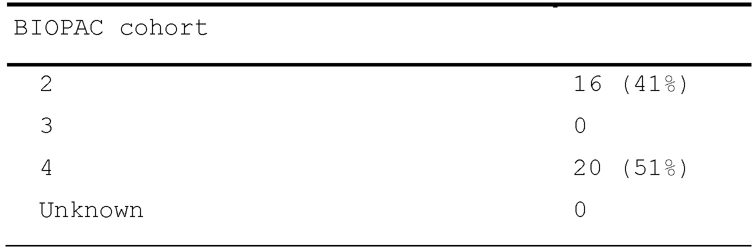

### Statistics

Comparison of PRO-C22 levels were made using a Kruskal-Wallis test followed by Dunn's multiple comparison test comparing each solid cancer type to the healthy control group. This was followed by testing diagnostic accuracy of PRO-C22 by area under the receiver operating characteristic (AUROC) curve. The BIOPAC cohort was used to evaluate associations with overall survival (OS) using the median as cut point to define a group with high PRO-C22 levels. Kaplan Meier curve analysis and univariate Cox regression analysis were used to evaluate associations between PRO-C22 and OS. Asterisks indicate the following significance levels: ** p<0.01; *** p<0.001; **** p<0.0001.

### Results

### PRO-C22 ELISA development

Through the ELISA development process, optimizing steps were made to find an appropriate assay buffer, incubation time and temperature as well as concentrations of antibody and peptides to give the best competitive setting for the assay. The competitive immunoassay PRO-C22 used a horseradish peroxidase labeled mAb (HRP-mAb) to quantify the amount of COL22 in serum samples. The assay included a standard peptide (GPPGPPGQCDPSQCAYFASLAARPGNVKGP (SEQ ID No. 16)) that by competing away the interaction between HRP-mAb and a biotinylated version of the peptide (Biotin-AARPGNVKGP (SEQ ID No. 15)) was used to quantify circulating amount of COL22 in serum. The specificity was tested using a truncated (AARPGNVKG (SEQ ID No. 4)) and elongated (AARPGNVKGPA (SEQ ID No. 3)) version of the peptide that did not show any competition for the HRP-mAb. A non-sense coater (PGMRGMPGSP-Biotin (SEQ ID No. 22)) and non-sense 30 AA standard peptide (PGMRGMPGSPGGPGSDGKPGPPGSQGESGR (SEQ ID No. 17)) was evaluated for the ability to interfere with the competition as well, none of them showing any effect (Figure 1). In contrast, both the ten AA sequence selection peptide (AARPGNVKGP (SEQ ID No. 1)) and the 30 AA sequence standard peptide (GPPGPPGQCDPSQCAYFASLAARPGNVKGP (SEQ ID No. 16)) successfully inhibited the interaction dose dependently (Figure 1).

The linear measurement range was determined to be 1.0-53.0ng/mL and LLOQ and ULOQ to be 1.95 and 125.0ng/mL, respectively. Through the intra- and inter- variation tests the precision of the assay was determined. Both the intra- and inter- variation was determined to be 3.6%. The minimum required dilution of serum was evaluated by the linearity of healthy serum samples two-fold diluted in the assay. This determined 1:2 (1+1) as minimum required dilution and limited the dilution to 1:4 (1+3) of healthy serum samples. This was based on a mean RE% of 103.7%.

The accuracy was determined by spiking recovery tests, where the mean RE% of peptide in serum was 105.1% and the mean RE% of serum in serum was 103.5%. The substances biotin, lipids and hemoglobin were tested for interference in different concentrations. The low concentrations of biotin of 5ng/ml were in three different samples found to have a mean RE% of 99.1 and the high concentration of 100ng/mL a mean RE% of 85.5%. The low lipid concentration of 1.5mg/mL in three serum samples had a mean RE% of 104.6% and the high concentration of 5mg/mL a mean RE% of 102.3%. The interference cut-off of hemoglobin was determined using concentrations varying by 0.5mg/mL of hemoglobin ranging from 0.50mg/mL to 5.0mg/mL. At 3.0mg/mL the mean RE% of three serum samples was 126.6% and exceeded the acceptable range of RE% (80-120%). Hence the cut-off was determined to be at a concentration of 2.5 mg/mL based on the mean RE% of 112.5%.

Stability was evaluated regarding both analyte and kit reagent stability. The mean RE% of three serum samples that underwent five freeze-thaw cycles was 103.1%. Serum samples incubated up to 48hrs at 4°C had a RE% of 107.2% and 106.1% for samples incubated up to 48hrs at 20°C. The kit reagents (buffer, biotinylated-peptide and HRP-mAb) were evaluated after 24hrs of incubation at 20°C. The stability of the reagents was evaluated by measuring the same ten samples used for intra- and inter- variation and calculating the RE%. The ten samples, measured in three individual runs, showed a mean RE% of 94.4%.

A summary of the technical validation is depicted in Table 3.

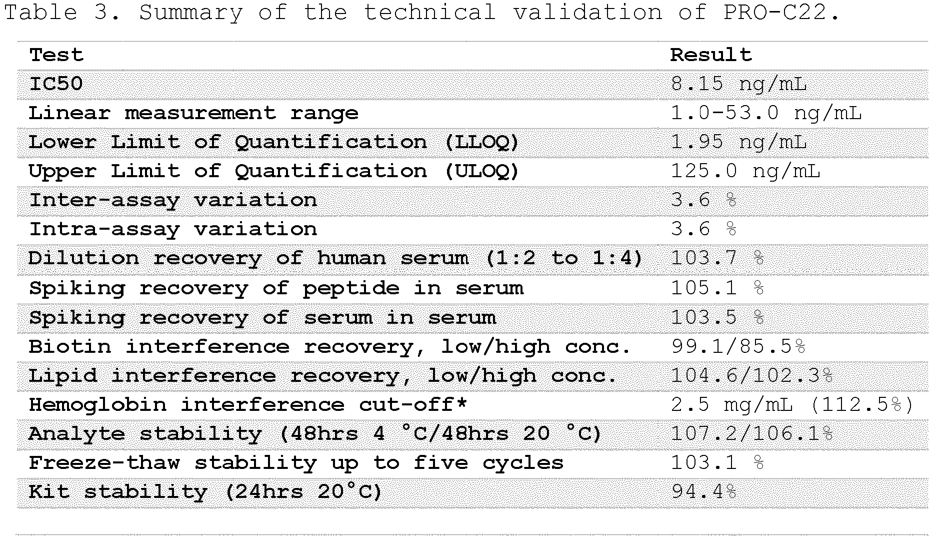

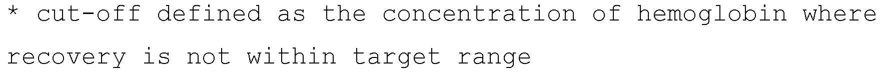

* cut-off defined as the concentration of hemoglobin where recovery is not within target range

### PRO-C22 in serum of cancer patients

In Cohort 1, PRO-C22 was significantly elevated in all solid cancer types compared to controls (Figure 2). This suggests that PRO-C22 i.e., circulating levels of COL22 was increased in serum from patients suffering from various solid tumor types. The area under the ROC curve (AUROC) was used to evaluate the ability of PRO-C22 to differentiate between cancer patients and healthy controls (Table 4).

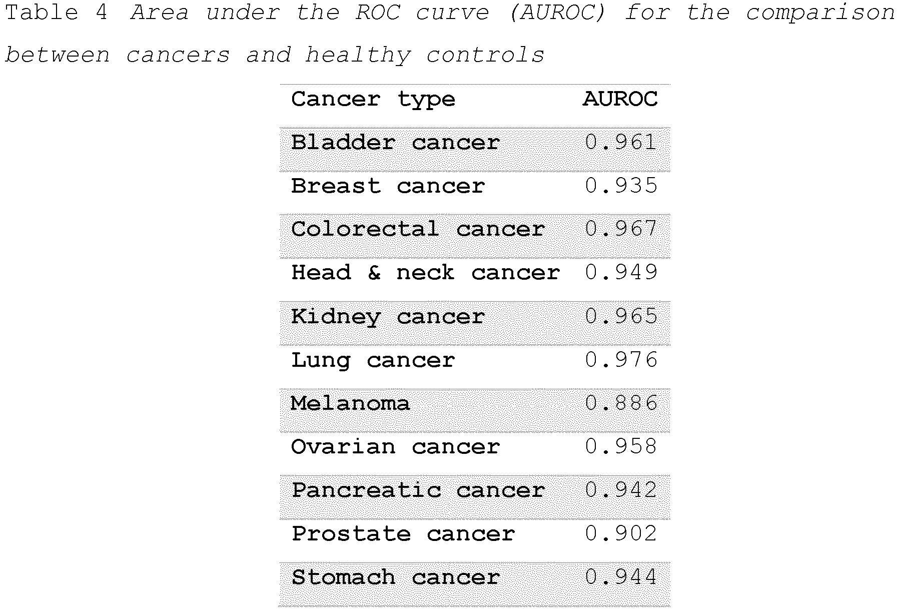

The range of AUROC varied from 0.886-0.976, which indicates that PRO-C22 discriminate excellently (AUROC greater than 0.8) between cancer patients and healthy controls. PRO-C22 was validated to be elevated in patients with pancreas cancer in the second (BIOPAC) cohort (Figure 3). When evaluating and association with overall survival (OS), high PRO-C22 (above the median: 50th percentile) was significantly associated with poor OS in pancreas cancer with a Hazard Ratio of 3.987 (95%CI: 1.724-8.806) compared to patients with low levels. Likewise, the median OS time was 162 days in PRO-C22-high patients and 1363 in PRO-C22-low patients (log rank p-value = 0.0011).

### Conclusion

In this study, an ELISA to quantify the presence of type XXII collagen in blood was successfully developed, optimized, and validated. The PRO-C22 ELISA was technically robust, accurate and sensitive. The levels of circulating type XXII collagen could be assessed in the serum of cancer patients and healthy controls with levels of PRO-C22 being significantly higher in all cancers tested compared to healthy controls. The levels of PRO-C22 may be used to distinguish between cancer patients and healthy controls, The levels of PRO-C22 may also be used to provide a prognosis for overall survival in pancreatic cancer. In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used to mean 'including or consisting of'. No acknowledgement of any prior published document herein should be taken to be an admission or representation that the teaching thereof was common general knowledge in Australia or elsewhere at the date hereof.

### References

[1] R. L. Siegel, K. D. Miller, H. E. Fuchs, and A. Jemal, "Cancer Statistics, 2021," CA. Cancer J. Clin., vol. 71, no. 1, pp. 7-33, Jan. 2021, doi: 10.3322/caac.21654.
[2] S. M. Hanash, C. S. Baik, and O. Kallioniemi, "Emerging molecular biomarkers-blood-based strategies to detect and monitor cancer," Nat. Rev. Clin. Oncol., vol. 8, no. 3, pp. 142-150, Mar. 2011, doi: 10.1038/nrclinonc.2010.220.
[3] M. Sund and R. Kalluri, "Tumor stroma derived biomarkers in cancer," Cancer Metastasis Rev., vol. 28, no. 1-2, pp. 177-183, Jun. 2009, doi: 10.1007/s10555-008-9175-2.
[4] D. Hanahan and R. A. Weinberg, "Hallmarks of Cancer: The Next Generation," Cell, vol. 144, no. 5, pp. 646-674, Mar. 2011, doi: 10.1016/j.cell.2011.02.013.
[5] N. Willumsen, L. B. Thomsen, C. L. Bager, C. Jensen, and M. A. Karsdal, "Quantification of altered tissue turnover in a liquid biopsy: a proposed precision medicine tool to assess chronic inflammation and desmoplasia associated with a pro-cancerous niche and response to immuno-therapeutic anti-tumor modalities," Cancer Immunol. Immunother., vol. 67, no. 1, pp. 1-12, Jan. 2018, doi: 10.1007/s00262-017-2074-z.
[6] N. I. Nissen, M. Karsdal, and N. Willumsen, "Collagens and Cancer associated fibroblasts in the reactive stroma and its relation to Cancer biology," J. Exp. Clin. Cancer Res., vol. 38, no. 1, p. 115, Dec. 2019, doi: 10.1186/s13046-019-1110-6.
[7] S. Xu et al., "The role of collagen in cancer: from bench to bedside," J. Transl. Med., vol. 17, no. 1, p. 309, Dec. 2019, doi: 10.1186/s12967-019-2058-1.
[8] L. M. Shaw and B. R. Olsen, "FACIT collagens: diverse molecular bridges in extracellular matrices," Trends Biochem. Sci., vol. 16, pp. 191-194, Jan. 1991, doi: 10.1016/0968-0004 (91) 90074-6.
[9] M. Koch et al., "A novel marker of tissue junctions, collagen XXII," J. Biol. Chem., vol. 279, no. 21, pp. 22514-22521, May 2004, doi: 10.1074/jbc.M400536200.
[10] Q. V. Ton et al., "Collagen COL22A1 maintains vascular stability and mutations in COL22A1 are potentially associated with intracranial aneurysms," Dis. Model. Mech., vol. 11, no. 12, Dec. 2018, doi: 10.1242/dmm.033654.
[11] K. MISAWA et al., "Prognostic value of type XXII and XXIV collagen mRNA expression in head and neck cancer patients," Mol. Clin. Oncol., vol. 2, no. 2, pp. 285-291, Mar. 2014, doi: 10.3892/mco.2013.233.
[12] T. Watanabe et al., "A Human Skin Model Recapitulates Systemic Sclerosis Dermal Fibrosis and Identifies COL22A1 as a TGFβ Early Response Gene that Mediates Fibroblast to Myofibroblast Transition," Genes (Basel)., vol. 10, no. 2, p. 75, Jan. 2019, doi: 10.3390/genes10020075.
[13] R. Bauer et al., "Inhibition of Collagen XVI Expression Reduces Glioma Cell Invasiveness," Cell. Physiol. Biochem., vol. 27, no. 3-4, pp. 217-226, 2011, doi: 10.1159/000327947.
[14] M. Koch et al., "α1(XX) Collagen, a New Member of the Collagen Subfamily, Fibril-associated Collagens with Interrupted Triple Helices," J. Biol. Chem., vol. 276, no. 25, pp. 23120-23126, Jun. 2001, doi: 10.1074/jbc.M009912200.
[15] B. Charvet et al., "Knockdown of col22a1 gene in zebrafish induces a muscular dystrophy by disruption of the myotendinous junction.," Development, vol. 140, no. 22, pp. 4602-4613, Nov. 2013, doi: 10.1242/dev.096024.
[16] A. Y. Wong and J. L. Whited, "Parallels between wound healing, epimorphic regeneration and solid tumors," Development, vol. 147, no. 1, Jan. 2020, doi: 10.1242/dev.181636.
[17] G. Manzo, "Similarities Between Embryo Development and Cancer Process Suggest New Strategies for Research and Therapy of Tumors: A New Point of View," Front. Cell Dev. Biol., vol. 7, Mar. 2019, doi: 10.3389/fcell.2019.00020.
[18] Kabat EA, Wu TT, Bilofsky H, Reid-Miller M, Perry H. Sequence of Proteins of Immunological Interest. Bathesda: National Institute of Health; 1983

## Claims

1. A monoclonal antibody that specifically binds to a C-terminus amino acid sequence AARPGNVKGP (SEQ ID No. 1) .

2. A monoclonal antibody as claimed in claim 1, wherein the monoclonal antibody does not specifically bind to an elongated version of said C-terminus amino acid sequence which is AARPGNVKGPA (SEQ ID No. 3) .

3. A monoclonal antibody as claimed in claim 1 or 2, wherein the monoclonal antibody does not specifically bind to a truncated version of said C-terminus amino acid sequence which is AARPGNVKG (SEQ ID No. 4) .

4. A monoclonal antibody as claimed in any one of claims 1 to 3, wherein the monoclonal antibody is raised against a synthetic peptide having the C-terminus amino acid sequence AARPGNVKGP (SEQ ID No. 1) .

5. A method of immunoassay for detecting and/or monitoring cancer in a patient, the method comprising:
i) contacting a sample from a patient with a monoclonal antibody as claimed in any one of claims 1 to 4; and
ii) detecting and determining the amount of binding between said monoclonal antibody and peptides in the sample.

6. The method of claim 5, wherein the method further comprises:
iii) correlating said amount of binding of said monoclonal antibody as determined in step (ii) with values associated with normal healthy subjects, and/or with values associated with known disease severity, and/or with values obtained from said patient at a previous time point, and/or with a predetermined cut-off value.

7. The method of claim 5 or 6, wherein the cancer is bladder cancer, breast cancer, colorectal cancer, head and neck cancer, kidney cancer, lung cancer, melanoma, ovarian cancer, pancreatic cancer, prostate cancer, or stomach cancer.

8. The method of claim 7, wherein the cancer is pancreatic cancer.

9. The method of claim 8 further comprising providing a prognosis of overall survival, wherein an amount of binding of said monoclonal antibody as determined in step (ii) above the median value associated with known pancreatic cancer patients is associated with poor overall survival.

10. The method of any one of claims 5 to 9, wherein the sample is a biofluid sample selected from blood, serum or plasma.

11. The method of any one of claims 5 to 10, wherein the immunoassay is a competition assay or a sandwich assay.

12. The method of any one of claims 5 to 11, wherein the immunoassay is a radio-immunoassay or an enzyme-linked immunosorbent assay.

13. An immunoassay kit comprising a monoclonal antibody of any one of claims 1 to 4 and at least one of;
- a streptavidin coated well plate;
- a biotinylated peptide Biotin-L-AARPGNVKGP (SEQ ID No. 15), wherein L is an optional linker;
- a secondary antibody for use in a sandwich immunoassay;
- a calibrator protein comprising the C-terminus amino acid sequence AARPGNVKGP (SEQ ID No. 1);
- an antibody biotinylation kit;
- an antibody HRP labelling kit;
- an antibody radiolabelling kit; and
- an assay visualisation kit.

## Patentansprüche

1. Monoklonaler Antikörper, der spezifisch an die C-terminale Aminosäuresequenz AARPGNVKGP (SEQ ID No. 1) bindet.

2. Monoklonaler Antikörper nach Anspruch 1, wobei der monoklonale Antikörper nicht spezifisch an eine verlängerte Version der C-terminalen Aminosäuresequenz bindet, die AARPGNVKGPA (SEQ ID No. 3) ist.

3. Monoklonaler Antikörper nach Anspruch 1 oder 2, wobei der monoklonale Antikörper nicht spezifisch an eine verkürzte Version der C-terminalen Aminosäuresequenz bindet, die AARPGNVKG (SEQ ID No. 4) ist.

4. Monoklonaler Antikörper nach einem der Ansprüche 1 bis 3, wobei der monoklonale Antikörper gegen ein synthetisches Peptid mit der C-terminalen Aminosäuresequenz AARPGNVKGP (SEQ ID Nr. 1) gezüchtet wird.

5. Immunassay-Verfahren zum Nachweisen und/oder Überwachen von Krebs bei einem Patienten, wobei das Verfahren umfasst:
i) Inkontaktbringen einer Probe eines Patienten mit einem monoklonalen Antikörper nach einem der Ansprüche 1 bis 4; und
ii) Nachweisen und Bestimmen der Menge der Bindung zwischen dem monoklonalen Antikörper und Peptiden in der Probe.

6. Verfahren nach Anspruch 5, wobei das Verfahren ferner umfasst:
iii) Korrelieren der in Schritt (ii) bestimmten Menge der Bindung des monoklonalen Antikörpers mit Werten, die normalen gesunden Probanden zugeordnet sind, und/oder mit Werten, die einer bekannten Krankheitsschwere zugeordnet sind, und/oder mit Werten, die von dem Patienten zu einem früheren Zeitpunkt erhalten wurden, und/oder mit einem vorbestimmten Cut-off-Wert.

7. Verfahren nach Anspruch 5 oder 6, wobei der Krebs Blasenkrebs, Brustkrebs, Darmkrebs, Kopf- und Halskrebs, Nierenkrebs, Lungenkrebs, Melanom, Eierstockkrebs, Bauchspeicheldrüsenkrebs, Prostatakrebs oder Magenkrebs ist.

8. Verfahren nach Anspruch 7, wobei der Krebs Bauchspeicheldrüsenkrebs ist.

9. Verfahren nach Anspruch 8, ferner umfassend ferner das Bereitstellen einer Prognose des Gesamtüberlebens, wobei eine in Schritt (ii) bestimmte Menge der Bindung des monoklonalen Antikörpers oberhalb des Medianwerts, der bekannten Bauchspeicheldrüsenkrebs-Patienten zugeordnet ist, einem schlechten Gesamtüberleben zugeordnet wird.

10. Verfahren nach einem der Ansprüche 5 bis 9, wobei die Probe eine Bioflüssigkeitsprobe ist, die aus Blut, Serum oder Plasma ausgewählt wird.

11. Verfahren nach einem der Ansprüche 5 bis 10, wobei der Immunassay ein Kompetitionsassay oder ein Sandwich-Assay ist.

12. Verfahren nach einem der Ansprüche 5 bis 11, wobei der Immunassay ein Radioimmunassay oder ein Enzyme-Linked Immunosorbent Assay ist.

13. Immunassay-Kit, umfassend einen monoklonalen Antikörper nach einem der Ansprüche 1 bis 4 und mindestens eines von:
- einer mit Streptavidin beschichteten Weil-Platte;
- einem biotinylierten Peptid Biotin-L-AARPGNVKGP (SEQ ID No. 15), wobei L ein optionaler Linker ist;
- einem sekundären Antikörper zur Verwendung in einem Sandwich-Immunassay;
- einem Kalibrierungsprotein, das die C-terminale Aminosäuresequenz AARPGNVKGP (SEQ ID Nr. 1) umfasst;
- einem Antikörper-Biotinylierungs-Kit;
- einem Antikörper-HRP-Markierungs-Kit;
- einem Antikörper-Radiomarkierungs-Kit; und
- einem Assay-Visualisierungs-Kit.

## Revendications

1. Anticorps monoclonal qui se lie spécifiquement à une séquence d'acides aminés C-terminale AARPGNVKGP (SEQ ID n° 1).

2. Anticorps monoclonal selon la revendication 1, l'anticorps monoclonal ne se liant pas spécifiquement à une version allongée de ladite séquence d'acides aminés C-terminale qui est AARPGNVKGPA (SEQ ID N° 3).

3. Anticorps monoclonal selon la revendication 1 ou 2, l'anticorps monoclonal ne se liant pas spécifiquement à une version tronquée de ladite séquence d'acides aminés C-terminale qui est AARPGNVKG (SEQ ID N° 4).

4. Anticorps monoclonal selon l'une quelconque des revendications 1 à 3, l'anticorps monoclonal étant dirigé contre un peptide synthétique présentant la séquence d'acides aminés C-terminale AARPGNVKGP (SEQ ID N° 1).

5. Procédé de dosage immunologique pour détecter et/ou surveiller un cancer chez un patient, le procédé comprenant :
i) la mise en contact d'un échantillon provenant d'un patient avec un anticorps monoclonal selon l'une quelconque des revendications 1 à 4 ; et
ii) la détection et la détermination de la quantité de liaison entre ledit anticorps monoclonal et des peptides dans l'échantillon.

6. Procédé selon la revendication 5, le procédé comprenant en outre :
iii) la corrélation de ladite quantité de liaison dudit anticorps monoclonal telle que déterminée à l'étape (ii) avec des valeurs associées à des sujets sains normaux et/ou avec des valeurs associées à une gravité connue de maladie et/ou avec des valeurs obtenues à partir dudit patient à un moment antérieur et/ou avec une valeur seuil statistique prédéterminée.

7. Procédé selon la revendication 5 ou 6, dans lequel le cancer est un cancer de la vessie, un cancer du sein, un cancer colorectal, un cancer de la tête et du cou, un cancer du rein, un cancer du poumon, un mélanome, un cancer de l'ovaire, un cancer du pancréas, un cancer de la prostate ou un cancer de l'estomac.

8. Procédé selon la revendication 7, dans lequel le cancer est le cancer du pancréas.

9. Procédé selon la revendication 8, comprenant en outre la fourniture d'un pronostic de survie globale, dans lequel une quantité de liaison dudit anticorps monoclonal, telle que déterminée à l'étape (ii), supérieure à la valeur médiane associée à des patients connus atteints d'un cancer du pancréas, est associée à une faible survie globale.

10. Procédé selon l'une quelconque des revendications 5 à 9, dans lequel l'échantillon est un échantillon de fluide biologique choisi parmi le sang, le sérum ou le plasma.

11. Procédé selon l'une quelconque des revendications 5 à 10, dans lequel le dosage immunologique est un dosage par compétition ou un dosage de type sandwich.

12. Procédé selon l'une quelconque des revendications 5 à 11, dans lequel le dosage immunologique est un dosage radio-immunologique ou un dosage d'immunoabsorption enzymatique.

13. Trousse de dosage immunologique comprenant un anticorps monoclonal selon l'une quelconque des revendications 1 à 4 et au moins l'un parmi :
- une plaque à puits revêtue de streptavidine ;
- un peptide biotinylé Biotine-L-AARPGNVKGP (SEQ ID n° 15), où L est un lieur facultatif ;
- un anticorps secondaire pour une utilisation dans un dosage de type sandwich ;
- une protéine étalon comprenant la séquence d'acides aminés C-terminale AARPGNVKGP (SEQ ID n° 1) ;
- un kit de biotinylation d'anticorps ;
- un kit de marquage HRP d'anticorps ;
- un kit de radiomarquage d'anticorps ; et
- un kit de visualisation de dosage.
